# EUROPEAN PATENT APPLICATION

(11) **EP 4 406 938 A1**
(43) Date of publication of application: **31.07.2024**
(21) Application number: 22872995.0
(22) Date of filing: 22.09.2022
(51) Int. Cl.: C07C 381/00, C07D 213/71, C07D 239/38

(54) **METHOD FOR PRODUCING TETRAFLUOROSULFANYL GROUP-CONTAINING ARYL COMPOUND**

(30) Priority: 22.09.2021 JP 2021154611; 19.10.2021 JP 2021171007; 23.03.2022 JP 2022046766
(71) Applicant: AGC INC., Chiyoda-ku, Tokyo 1008405 (JP); The University of Tokyo, Bunkyo-ku, Tokyo 113-8654 (JP)
(72) Inventor: OKAZOE Takashi, Tokyo 100-8405 (JP); AIKAWA Kohsuke, Tokyo 113-8654 (JP); NOZAKI Kyoko, Tokyo 113-8654 (JP); YASUO Eisuke, Tokyo 113-8654 (JP)
(74) Representative: Müller-Boré & Partner Patentanwälte PartG mbB
(86) International application number: PCT/JP2022/035414
(87) International publication number: WO 2023/048244

(57) **Abstract**

The present invention is a method for producing a tetrafluorosulfanyl group-containing aryl compound, in which a thioaryl compound represented by General Formula (2) is subjected to a reaction by adding to an olefin compound represented by General Formula (3) in the presence of a radical initiator to synthesize a tetrafluorosulfanyl group-containing aryl compound represented by General Formula (1) [in the formula, A¹ is an aryl group which may have a substituent, or a heteroaryl group which may have a substituent; and R¹, R², R³, and R⁴ are each independently a hydrogen atom, a fluorine atom, or a chlorine atom, and two or more of R¹, R², R³, and R⁴ are a fluorine atom].

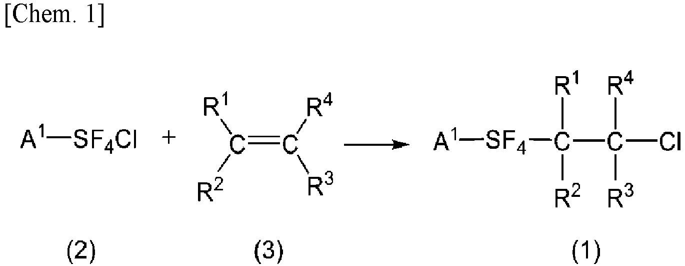

## Description

### [Technical Field]

The present invention relates to a method for producing a tetrafluorosulfanyl group-containing aryl compound in which a tetrafluorosulfanyl group is introduced into an aryl group.

Priority is claimed on Japanese Patent Application No. 2021-154611, filed September 22, 2021, Japanese Patent Application No. 2021-171007, filed October 19, 2021, and Japanese Patent Application No. 2022-046766 filed March 23, 2022, the contents of which are incorporated herein by reference.

### [Background Art]

A fluorine-containing compound exhibits unique material characteristics and unique biological activities. For example, in a case where a fluorine atom or a trifluoromethyl group is introduced into an organic compound, hydrophobicity is improved in addition to metabolic stability. For this reason, the use of compounds containing fluorine atoms is prominent, particularly in the development of medicines and agricultural chemicals. In recent years, from the viewpoint of improving the functionality of fluorine-containing compounds, the focus of research has shifted to functional groups that have a larger effect on improving hydrophobicity. For example, a sulfur-fluorine functional group having a structure in which a plurality of fluorine atoms are bonded to hexavalent sulfur has attracted attention due to the fact that it imparts high hydrophobicity compared with the corresponding carbon-fluorine functional group (Non-Patent Document 1).

On the other hand, it is difficult to introduce a sulfur-fluorine functional group into an existing compound. For this reason, little progress has been made in the use of a sulfur-fluorine functional group in active ingredients of pharmaceutical drugs or agricultural chemicals or in organic materials despite the high appeal thereof. Research that has progressed so far relates to a study of a method of introducing a pentafluoro-λ⁶-sulfanyl (SF₅) group as a sulfur-fluorine functional group. As a practical synthesis method for an aromatic SF₅ compound, two-step synthesis using a chlorotetrafluoro-λ⁶-sulfanyl (SF₄Cl) compound as an intermediate has been developed (Patent Document 1). In addition, an aromatic SF₄CF₃ compound has been synthesized and has been revealed to have greater hydrophobicity than the aromatic SF₅ compound (Non-Patent Document 2).

### [Citation List]

### [Patent Document]

[Patent Document 1] PCT International Publication No. WO2010/014665

### [Non-Patent Document]

[Non-patent Document 1] Savoie and Welch, Chemical Reviews, 2015, vol.115, p.1130-1190.
[Non-patent document 2] Kirsch and Hahn, Eur. J. Org. Chem. 2006, vol.2006(5), p.1125-1131.

### [Summary of Invention]

### [Technical Problem]

An object of the present invention is to provide a novel manufacturing method that makes it possible to efficiently synthesize a tetrafluorosulfanyl group-containing aryl compound in which a tetrafluorosulfanyl group is introduced into an aryl group.

### [Solution to Problem]

The inventors of the present invention found that in a case of reacting a SF₄Cl compound with a fluorine-substituted olefin in the presence of a radical initiator, an aryl compound containing a tetrafluorosulfanyl group can be synthesized in a single step, thereby completing the present invention.

That is, the present invention is as follows.
[1] A method for producing a tetrafluorosulfanyl group-containing aryl compound, comprising:
   subjecting a thioaryl compound represented by General Formula (2) to a reaction by adding to an olefin compound represented by General Formula (3) in the presence of a radical initiator to synthesize a tetrafluorosulfanyl group-containing aryl compound represented by General Formula (1).
   [Chem. 1]

   A¹-SF₄Cl (2)

   [In the formula, A¹ is an aryl group which may have a substituent, or a heteroaryl group which may have a substituent.] 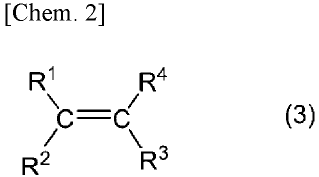
   [In the formula, R¹, R², R³, and R⁴ are each independently a hydrogen atom, a fluorine atom, or a chlorine atom, provided that two or more of R¹, R², R³, and R⁴ are a fluorine atom.] 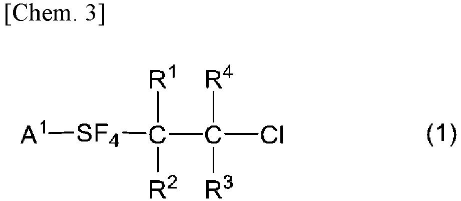
   [In the formula, A¹, R¹, R², R³, and R⁴ are the same as above.]
[2] The method for producing a tetrafluorosulfanyl group-containing aryl compound according to [1], in which A¹ is an aryl which may have one or more kinds of substituents selected from the group consisting of a halogen atom, an alkyl group, an alkenyl group, an aryl group, an alkoxy group, a hydroxy group, a carboxy group, an acyl group, a cyano group, an amino group, and a nitro group.
[3] The method for producing a tetrafluorosulfanyl group-containing aryl compound according to [1] or [2], in which the reaction is carried out at -40°C to 130°C.
[4] A tetrafluorosulfanyl group-containing aryl compound represented by General Formula (1). 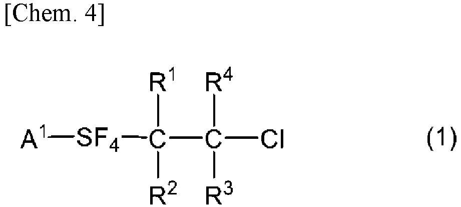 [In the formula, A¹ is an aryl group which may have a substituent, or a heteroaryl group which may have a substituent; and R¹, R², R³, and R⁴ are each independently a hydrogen atom, a fluorine atom, or a chlorine atom, and two or more of R¹, R², R³, and R⁴ are a fluorine atom.]

### [Advantageous Effects of Invention]

According to the method according to the present invention, an addition reaction of a SF₄Cl compound to a fluorine-substituted olefin can be carried out in a single step, and thus a tetrafluorosulfanyl group-containing aryl compound can be efficiently synthesized.

### [Description of Embodiments]

In the present invention and the present specification, "Cₚ₁₋ₚ₂" (p1 and p2 are each a positive integer satisfying p1 < p2) means a group in which the number of carbon atoms is p1 to p2.

In the present invention and the present specification, a "C₁₋₆ alkyl group" is an alkyl group having 1 to 6 carbon atoms, and it may be linear or may be branched. Examples of the C₁₋₆ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, and a hexyl group.

In the present invention and the present specification, the term "C₁₋₆ alkoxy group" refers to a group in which an oxygen atom is bonded to the bond terminal of a C₁₋₆ alkyl group. The C₁₋₆ alkoxy group may be linear or may be branched. Examples of the C₁₋₆ alkoxy group include a methoxy group, an ethoxy group, a propoxy group, a butoxy group, a tert-butoxy group, a pentyloxy group, and a hexyloxy group.

In the present invention and the present specification, the term "C₂₋₆ alkenyl group" refers to a group in which at least one carbon-carbon bond of an alkyl group having 2 to 6 carbon atoms is an unsaturated bond. The C₂₋₆ alkenyl group may be linear or may be branched. Examples of the C₂₋₆ alkenyl group include a vinyl group, an allyl group, a butenyl group, a pentenyl group, and a hexenyl group.

In the present invention and the present specification, "C₂₋₇ acyl group" refers to a group in which a hydrocarbon group moiety obtained by removing a carbonyl group from an acyl group is a C₁₋₆ alkyl group, a C₂₋₆ alkenyl group, an aryl group of a 5-membered ring or 6-membered ring, or a heteroaryl group of a 5-membered ring or 6-membered ring. The hydrocarbon group moiety of the acyl group may be linear or may be branched. Examples of the C₂₋₇ acyl group include a formyl group, an acetyl group, a propanoyl group, a propenoyl group, and a benzoyl group.

In the present invention and the present specification, the term "halogen atom" refers to a fluorine atom, a chlorine atom, a bromine atom, or an iodine atom, and the term "halogen atom other than a fluorine atom" refers to a chlorine atom, a bromine atom, or an iodine atom. As an example of the "halogen atom other than a fluorine atom," a chlorine atom or a bromine atom is preferable, and a chlorine atom is particularly preferable.

In addition, hereinafter, "compound (n)" means a compound represented by Formula (n).

### <Addition reaction between SF₄Cl compound and fluorine-substituted olefin>

In a method for producing a tetrafluorosulfanyl group-containing aryl compound (hereinafter, sometimes referred to as "SF₄-containing aryl compound") according to the present invention, the sulfur atom in the SF₄Cl group in the SF₄Cl group-containing aryl compound is added to a fluorine-substituted olefin. Specifically, a thioaryl compound represented by General Formula (2) is subjected to a reaction by adding to an olefin compound represented by General Formula (3) in the presence of a radical initiator. As a result, a tetrafluorosulfanyl group-containing aryl compound (1) having higher hydrophobicity than a SFs-containing aryl compound is obtained.

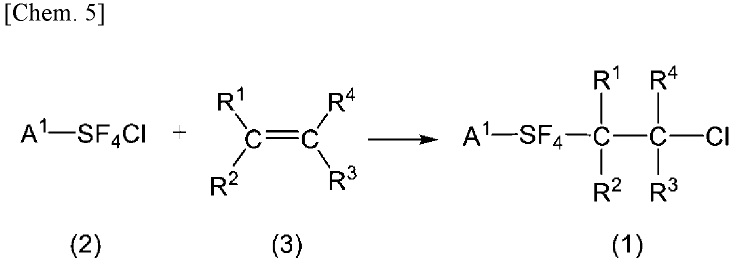

In General Formula (2) and General Formula (1), A¹ is an aryl group which may have a substituent, or a heteroaryl group which may have a substituent. The aryl group is not particularly limited. Examples thereof include a phenyl group, a naphthyl group, an anthryl group, and a 9-fluorenyl group, where a phenyl group is particularly preferable. The heteroaryl group is not particularly limited, and examples thereof include a pyridyl group, a pyrazinyl group, a pyrimidinyl group, a pyridazinyl group, a pyrazolyl group, a quinolyl group, an isoquinolyl group, a pyrrolyl group, an imidazolyl group, an indolyl group, a furyl group, a benzofuryl group, a thienyl group, a benzothienyl group, an oxazolyl group, an isoxazolyl group, a thiazolyl group, and an isothiazolyl group.

"The aryl group which may be substituted" is a group in which one or more or a plurality of hydrogen atoms bonded to carbon atoms of an aryl group, preferably 1 to 3 hydrogen atoms, are substituted with other functional groups. Similarly, "the heteroaryl group which may be substituted" is a group in which one or more or a plurality of hydrogen atoms bonded to carbon atoms of a heteroaryl group, preferably 1 to 3 hydrogen atoms, are substituted with other functional groups. In a case where two or more substituents are contained, the substituents may be the same or different from each other.

The aryl group and heteroaryl group as A¹ may have one or two or more substituents in addition to the sulfur atom for fluorination. Examples of the substituent include a halogen atom, an alkyl group, an alkenyl group, an alkoxy group, an aryl group, an acyl group, a hydroxy group, a carboxyl group, an alkyloxycarbonyl group, an aryloxycarbonyl group, a cyano group, and an amino group. The alkyl group is preferably a C₁₋₆ alkyl group, the alkenyl group is preferably a C₂₋₆ alkyl group, the alkoxy group is preferably a C₁₋₆ alkoxy group, and the acyl group is preferably a C₂₋₇ acyl group. The alkyloxycarbonyl group is preferably a group in which the alkyl group moiety is a C₁₋₆ alkyl group, and more preferably a group in which the alkyl group moiety is a C₁₋₃ alkyl group. The aryloxycarbonyl group is preferably a group in which the aryl group moiety is a phenyl group.

In General Formula (3), R¹, R², R³, and R⁴ are each independently a hydrogen atom, a fluorine atom, or a chlorine atom. However, two or more of R¹, R², R³, and R⁴ are a fluorine atom. Examples of the compound (3) include CF₂=CF₂, CF₂=CFCl, CF₂=CHF, CF₂=CCl₂, CF₂=CHCl, CF₂=CH₂, CFCl=CFCl, CFCl=CHF, and CHF=CHF.

The amount of the thioaryl compound (2) that is added to a reaction system may be any amount that is equal to or larger than the stoichiometric amount thereof. In terms of reaction efficiency and cost, the amount of the thioaryl compound (2) to be used is preferably 1 to 10 equivalents and more preferably 1 to 6 equivalents of the olefin compound (3).

The addition reaction between the thioaryl compound (2) and the olefin compound (3) was carried out in the presence of a radical initiator. As the radical initiator, an initiator known in the related art can be used. Specifically, for example, an azo compound or a peroxide can be used as the radical initiator. The radical initiator to be used in the addition reaction between the thioaryl compound represented by General Formula (2) and the olefin compound represented by General Formula (3) preferably includes an azo compound.

Examples of the radical initiators as the azo compound include azobisisobutyronitrile (hereinafter, also referred to as "AIBN"), 2,2'-azobis-2-methylbutyronitrile, 2,2'-azobis-2,4-dimethylvaleronitrile (ADVN), 2,2'-azobis-N-butyl-2-methylpropionamide, dimethyl-2,2'-azobis-2-methylpropionamide, and 1,1'-azobiscyclohexane-1-carbonitrile. In terms of solubility in a reaction system, AIBN, 2,2'-azobis-2-methylbutyronitrile, or 2,2'-azobis-2,4-dimethylvaleronitrile is preferable.

Examples of the radical initiator as the peroxide include benzoyl peroxide, t-butyl peroxide, acetyl peroxide, diisopropyl peroxydicarbonate, t-butylperoxy-2-ethylhexanate, 2-hexyl-peroxy-2-ethylhexanate, and 1,1,3,3-tetramethylbutylperoxy-2-ethylhexanoate. In terms of easy control of the reaction, benzoyl peroxide, t-butyl peroxide, t-butylperoxy-2-ethylhexanate, or 2-hexyl-peroxy-2-ethylhexanate is preferable.

The addition reaction between the thioaryl compound (2) and the olefin compound (3) can be carried out in a solvent inert to the addition reaction. The inert solvent is not particularly limited; however, it is preferably an aprotic polar solvent. Examples of the aprotic polar solvent include acetonitrile (MeCN), N,N'-dimethylformamide (DMF), N,N-dimethylacetamide, dimethyl sulfoxide (DMSO), tetrahydrofuran (THF), dichloromethane (DCM), 1,2-dichloroethane (DCE), and diethyl ether. The solvent to be used in the reaction may be a mixed solvent of two or more kinds of solvents.

In the addition reaction, a reaction solution obtained by mixing the thioaryl compound (2), the olefin compound (3), and a radical initiator in a reaction solvent is reacted at an appropriate temperature for an appropriate time. The addition reaction proceeds under mild conditions. For example, the reaction temperature is not particularly limited as long as the reaction solvent is liquid. The reaction can be carried out at -40°C to 130°C, preferably carried out at 0°C to 80°C, and preferably carried out at 20°C to 50°C, and it can also be carried out at room temperature (0°C to 30°C). For example, in a case where the addition reaction is carried out at 20°C to 50°C for less than 3 hours, the target tetrafluorosulfanyl group-containing aryl compound (1) can be obtained in an intrinsically quantitative yield.

### [Examples]

Hereinafter, the present invention will be described in detail according to Examples; however, the present invention is not limited to these Examples.

The NMR apparatus used for the analysis in Examples and Comparative Examples is JNM-ECZ400S (400 MHz) manufactured by JEOL Ltd., and a reference value of tetramethylsilane is 0 PPM for ¹H NMR, and a reference value of C₆F₆ is -162 PPM for ¹⁹F NMR.

### <Synthesis of aryl compound containing tetrafluorosulfanyl group>

A tetrafluorosulfanyl group-containing aryl compound was synthesized according to the following synthetic procedure.

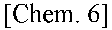

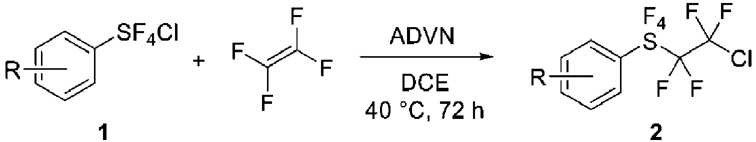

Under a nitrogen atmosphere, a solution obtained by dissolving the compound (1) (0.20 mmol) and ADVN (10% by mole) in DCE (0.1 M) was stirred in a microwave vial (10 mL) and then cooled to 0°C. Next, tetrafluoroethylene (TFE) was added to the solution (1.0 atm) by bubbling. After carrying out stirring in an oil bath at 40°C for 72 hours, insoluble solids were removed by filtration, and the filtrate was subjected to evaporation under reduced pressure. The obtained crude product was purified by flash silica gel column chromatography (n-hexane/EtOAc = 9/1 (volume ratio)) to obtain the desired compound (2).

### [Example 1]

### Synthesis of (2-chlorotetrafluoroethyltetrafluoro-λ⁶-sulfanyl)benzene

According to the procedure described above, (2-chlorotetrafluoroethyltetrafluoro-λ⁶-sulfanyl)benzene was obtained from (chlorotetrafluoro-λ⁶-sulfanyl)benzene. Following purification carried out by flash silica gel column chromatography (n-hexane/EtOAc = 9/1 (volume ratio)), (2-chlorotetrafluoroethyltetrafluoro-λ⁶-sulfanyl)benzene (0.024 g, yield: 37%) was obtained as colorless crystals.

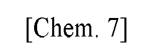

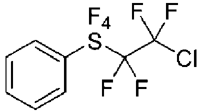

¹H NMR (400 MHz, CDCl₃) δ 7.71-7.83 (m, 2H), 7.42-7.54 (m, 3H).

¹⁹F NMR (376 MHz, CDCl₃) δ 47.7 (m, 4F), -67.9 (t, J = 10.8 Hz, 2F), -90.8 (m, 2F).

### [Example 2]

### Synthesis of 4-(2-chlorotetrafluoroethyltetrafluoro-λ⁶-sulfanyl)nitrobenzene

According to the procedure described above, 4-(2-chlorotetrafluoroethyltetrafluoro-λ⁶-sulfanyl)nitrobenzene was obtained from 4-(chlorotetrafluoro-λ⁶-sulfanyl)nitrobenzene. Following purification carried out by flash silica gel column chromatography (n-hexane/EtOAc = 9/1 (volume ratio)), 4-(2-chlorotetrafluoroethyltetrafluoro-λ⁶-sulfanyl)nitrobenzene (0.040 g, yield: 55%) was obtained as colorless crystals.

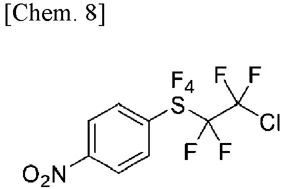

¹H NMR (400 MHz, CDCl₃) δ 8.33 (d, J = 8.7 Hz, 2H), 8.00 (d, J = 9.2 Hz, 2H).

¹³C NMR (100 MHz, CDCl₃) δ 159.4 (quint, J_{C-F} = 21.9 Hz), 149.2, 127.7 (quint, J_{C-F} = 4.8 Hz), 124.2, 122.1 (tt, J_{C-F} = 303.4, 35.6 Hz), 121.3 (ttquint, J_{C-F} = 312.1, 37.6, 36.6 Hz).

¹⁹F NMR (376 MHz, CDCl₃) δ 48.0 (m, 4F), -68.1 (t, J = 10.7 Hz, 2F), -90.7 (m, 2F).

### [Example 3]

### Synthesis of 3-(2-chlorotetrafluoroethyltetrafluoro-λ⁶-sulfanyl)nitrobenzene

According to the procedure described above, 3-(2-chlorotetrafluoroethyltetrafluoro-λ⁶-sulfanyl)nitrobenzene was obtained from 3-(chlorotetrafluoro-λ⁶-sulfanyl)nitrobenzene. Following purification carried out by flash silica gel column chromatography (n-hexane/EtOAc = 9/1 (volume ratio)), 3-(2-chlorotetrafluoroethyltetrafluoro-λ⁶-sulfanyl)nitrobenzene (0.034 g, yield: 46%) was obtained as a colorless liquid.

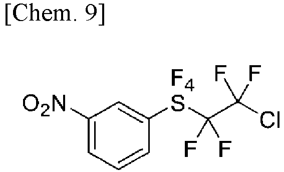

¹H NMR (400 MHz, CDCl₃) δ 8.67 (s, 1H), 8.41 (d, J = 8.2 Hz, 1H), 8.13 (d, J = 8.2 Hz, 1H), 7.71 (t, J = 8.2 Hz, 1H).

¹⁹F NMR (376 MHz, CDCl₃) δ 48.3 (m, 4F), -68.1 (t, J = 10.8 Hz, 2F), -90.6 (m, 2F).

### [Example 4]

### Synthesis of 4-(2-chlorotetrafluoroethyltetrafluoro-λ⁶-sulfanyl)fluorobenzene

According to the procedure described above, 4-(2-chlorotetrafluoroethyltetrafluoro-λ⁶-sulfanyl)fluorobenzene was obtained from 4-(chlorotetrafluoro-λ⁶-sulfanyl)fluorobenzene. Following purification carried out by flash silica gel column chromatography (n-hexane/EtOAc = 9/1 (volume ratio)), 4-(2-chlorotetrafluoroethyltetrafluoro-λ⁶-sulfanyl)fluorobenzene (0.035 g, yield: 51%) was obtained as a colorless liquid.

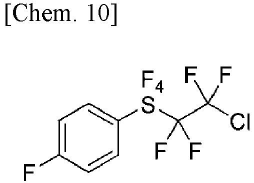

¹H NMR (400 MHz, CDCl₃) δ 7.77-7.82 (m, 2H), 7.11-7.16 (m, 2H).

¹⁹F NMR (376 MHz, CDCl₃) δ 49.0 (m, 4F), -68.0 (t, J = 10.8 Hz, 2F), -90.7 (m, 2F).

### [Example 5]

### Synthesis of 3-(2-chlorotetrafluoroethyltetrafluoro-λ⁶-sulfanyl)fluorobenzene

According to the procedure described above, 3-(2-chlorotetrafluoroethyltetrafluoro-λ⁶-sulfanyl)fluorobenzene was obtained from 3-(chlorotetrafluoro-λ⁶-sulfanyl)fluorobenzene. Following purification carried out by flash silica gel column chromatography (n-hexane/EtOAc = 9/1 (volume ratio)), 3-(2-chlorotetrafluoroethyltetrafluoro-λ⁶-sulfanyl)fluorobenzene (0.012 g, yield: 18%) was obtained as a colorless liquid.

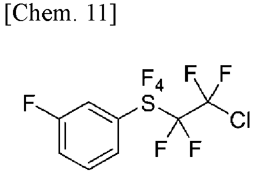

¹H NMR (400 MHz, CDCl₃) δ 7.58-7.61 (m, 1H), 7.50-7.55 (m, 1H), 7.42-7.49 (m, 1H), 7.20-7.26 (m, 1H).

¹⁹F NMR (376 MHz, CDCl₃) δ 48.0 (m, 4F), -68.0 (t, J = 10.8 Hz, 2F), -90.8 (m, 2F).

### [Example 6]

### Synthesis of 2-(2-chlorotetrafluoroethyltetrafluoro-λ⁶-sulfanyl)fluorobenzene

According to the procedure described above, 2-(2-chlorotetrafluoroethyltetrafluoro-λ⁶-sulfanyl)fluorobenzene was obtained from 2-(chlorotetrafluoro-λ⁶-sulfanyl)fluorobenzene. Following purification carried out by flash silica gel column chromatography (n-hexane/EtOAc = 9/1 (volume ratio)), 2-(2-chlorotetrafluoroethyltetrafluoro-λ⁶-sulfanyl)fluorobenzene (0.009 g, yield: 13%) was obtained as a colorless liquid.

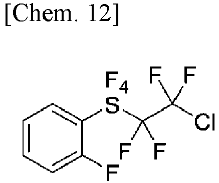

¹H NMR (400 MHz, CDCl₃) δ 7.79 (m, 1H), 7.51 (m, 1H), 7.19-7.26 (m, 2H).

¹⁹F NMR (376 MHz, CDCl₃) δ 49.0 (m, 4F), -68.0 (t, J = 10.8 Hz, 2F), -90.7 (m, 2F).

### [Example 7]

### Synthesis of 4-(2-chlorotetrafluoroethyltetrafluoro-λ⁶-sulfanyl)chlorobenzene

According to the procedure described above, 4-(2-chlorotetrafluoroethyltetrafluoro-λ⁶-sulfanyl)chlorobenzene was obtained from 4-(chlorotetrafluoro-λ⁶-sulfanyl)chlorobenzene. Following purification carried out by flash silica gel column chromatography (n-hexane/EtOAc = 9/1 (volume ratio)), 4-(2-chlorotetrafluoroethyltetrafluoro-λ⁶-sulfanyl)chlorobenzene (0.035 g, yield: 50%) was obtained as a colorless liquid.

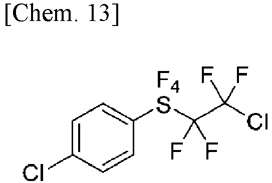

¹H NMR (400 MHz, CDCl₃) δ 7.72 (d, J = 9.1 Hz, 2H), 7.43 (d, J = 9.1 Hz, 2H).

¹⁹F NMR (376 MHz, CDCl₃) δ 48.4 (m, 4F), -68.0 (t, J = 10.8 Hz, 2F), -90.7 (m, 2F).

### [Example 8]

### Synthesis of 4-(2-chlorotetrafluoroethyltetrafluoro-λ⁶-sulfanyl)bromobenzene

According to the procedure described above, 4-(2-chlorotetrafluoroethyltetrafluoro-λ⁶-sulfanyl)bromobenzene was obtained from 4-(chlorotetrafluoro-λ⁶-sulfanyl)bromobenzene. Following purification carried out by flash silica gel column chromatography (n-hexane/EtOAc = 9/1 (volume ratio)), 4-(2-chlorotetrafluoroethyltetrafluoro-λ⁶-sulfanyl)bromobenzene (0.019 g, yield: 24%) was obtained as a colorless liquid.

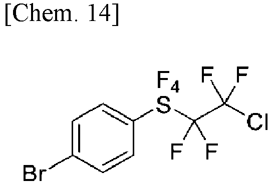

¹H NMR (400 MHz, CDCl₃) δ 7.65 (d, J = 9.1 Hz, 2H), 7.60 (d, J = 9.1 Hz, 2H).

¹⁹F NMR (376 MHz, CDCl₃) δ 48.3(m, 4F), -68.0 (t, J = 10.8 Hz, 2F), -90.8 (m, 2F).

### [Example 9]

### Synthesis of ethyl 4-(2-chlorotetrafluoroethyltetrafluoro-λ⁶-sulfanyl) benzoate

According to the procedure described above, ethyl 4-(2-chlorotetrafluoroethyltetrafluoro-λ⁶-sulfanyl) benzoate was obtained from ethyl 4-(chlorotetrafluoro-λ⁶-sulfanyl) benzoate. Following purification carried out by flash silica gel column chromatography (n-hexane/EtOAc = 9/1 (volume ratio)), ethyl 4-(2-chlorotetrafluoroethyltetrafluoro-λ⁶-sulfanyl) benzoate (0.038 g, yield: 48%) was obtained as a colorless liquid.

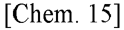

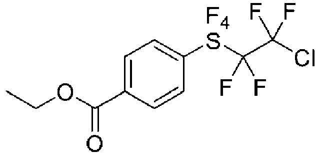

¹H NMR (400 MHz, CDCl₃) δ 8.12 (d, J = 8.7 Hz, 2H), 7.85 (d, J = 9.0 Hz, 2H), 4.42 (q, J = 7.0 Hz, 2H), 1.41 (d, J = 7.1 Hz, 3H).

¹⁹F NMR (376 MHz, CDCl₃) δ 47.6 (m, 4F), -68.0 (t, J = 10.8 Hz, 2F), -90.8 (m, 2F).

### [Example 10]

### Synthesis of 4-(2-chlorotetrafluoroethyltetrafluoro-λ⁶-sulfanyl)tert-butylbenzene

According to the procedure described above, 4-(2-chlorotetrafluoroethyltetrafluoro-λ⁶-sulfanyl)tert-butylbenzene was obtained from 4-(chlorotetrafluoro-λ⁶-sulfanyl)tert-butylbenzene. Following purification carried out by flash silica gel column chromatography (n-hexane/EtOAc = 9/1 (volume ratio)), 4-(2-chlorotetrafluoroethyltetrafluoro-λ⁶-sulfanyl)tert-butylbenzene (0.014 g, yield: 18%) was obtained as a colorless liquid.

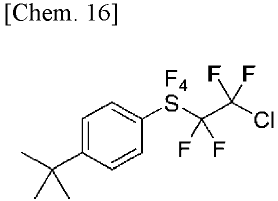

¹H NMR (400 MHz, CDCl₃) δ 7.70 (d, J = 9.1 Hz, 2H), 7.45 (d, J = 7.8 Hz, 2H), 1.34 (s, 9H).

¹⁹F NMR (376 MHz, CDCl₃) δ48.2 (m, 4F), -67.9 (t, J = 10.8 Hz, 2F), -90.8 (m, 2F).

### [Example 11]

### Synthesis of 2-(2-chlorotetrafluoroethyltetrafluoro-λ⁶-sulfanyl)-5-nitropyridine

According to the procedure described above, 2-(2-chlorotetrafluoroethyltetrafluoro-λ⁶-sulfanyl)-5-nitropyridine was obtained from 2-(chlorotetrafluoro-λ⁶-sulfanyl)-5-nitropyridine. Following purification carried out by flash silica gel column chromatography (n-hexane/EtOAc = 9/1 (volume ratio)), 2-(2-chlorotetrafluoroethyltetrafluoro-λ⁶-sulfanyl)-5-nitropyridine (0.024 g, yield: 33%) was obtained as a colorless liquid.

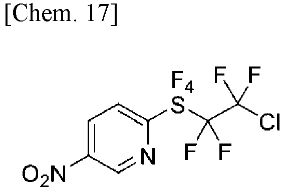

¹H NMR (400 MHz, CDCl₃) δ 9.39 (d, J = 2.7 Hz, 1H), 8.72 (m, 1H), 8.02 (d, J = 8.7 Hz, 1H).

¹⁹F NMR (376 MHz, CDCl₃) δ 38.5 (m, 4F), -68.3 (m, 2F), -91.2 (m, 2F).

### [Synthesis example 1]

Under a nitrogen atmosphere, (2-chlorotetrafluoroethyltetrafluoro-λ⁶-sulfanyl)benzene (0.07 mmol), AIBN (50% by mole), and tris(trimethylsilyl)silane (3.0 equivalents) were dissolved in toluene (1.3 mL) in a glass vial (15 mL), and stirring was carried out in an oil bath at 100°C for 1 hour. Thereafter, the solvent was evaporated under reduced pressure, and the obtained crude product was purified by flash silica gel column chromatography (n-hexane/EtOAc = 9/1 (volume ratio)), whereby the desired (1,1,2,2-tetrafluoroethyltetrafluoro-λ⁶-sulfanyl)benzene (0.014 g, yield: 40%) was obtained as a colorless liquid.

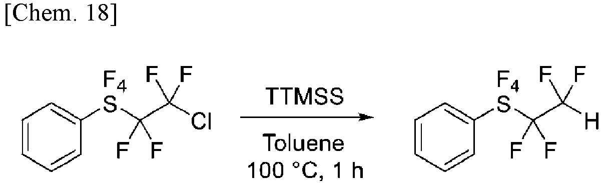

¹H NMR (400 MHz, CDCl₃) δ 7.76-7.81 (m, 2H), 7.44-7.51 (m, 3H), 6.18 (m, 1H).

¹⁹F NMR (376 MHz, CDCl₃) δ 42.7 (m, 4F), -98.0 (m, 2F), -134.3 (m, 2F).

### [Example 12]

### Synthesis of 4-(2-chlorotetrafluoroethyltetrafluoro-λ⁶-sulfanyl)aniline

To a solution obtained by dissolving 4-(2-chlorotetrafluoroethyltetrafluoro-λ⁶-sulfanyl)nitrobenzene (0.98 mmol) in ethanol (20 mL), iron (11 equivalents) and a saturated aqueous ammonium chloride solution (5 mL) were added, and stirring was carried out in an oil bath at 80°C for 2 hours. Thereafter, insoluble solids were removed by filtration through celite, and the filtrate was subjected to evaporation under reduced pressure. The obtained crude product was dissolved in ethyl acetate and washed with water, and the aqueous layer was extracted twice with ethyl acetate. Then, the combined organic extracts were dried with sodium sulfate, and the solvent was evaporated under reduced pressure. The obtained crude product was purified by flash silica gel column chromatography (n-hexane/EtOAc = 4/1 (volume ratio)), whereby the desired 4-(2-chlorotetrafluoroethyltetrafluoro-λ⁶-sulfanyl)aniline (0.311g, yield: 95%) was obtained as a pale yellow liquid.

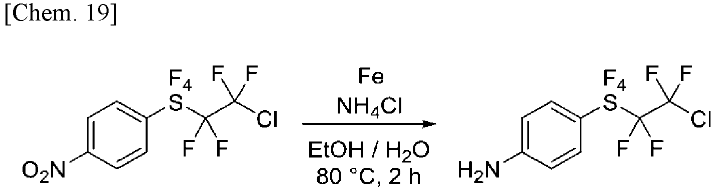

¹H NMR (400 MHz, CDCl₃) δ 7.55 (d, J = 8.7 Hz, 2H), 6.58 (d, J = 9.1 Hz, 2H), 3.97 (br, 2H)

¹⁹F NMR (376 MHz, CDCl₃) δ 49.5 (m, 4F), -67.9 (t, J = 10.8 Hz, 2F), -90.7 (m, 2F).

### [Example 13]

A tetrafluorosulfanyl group-containing aryl compound was synthesized according to the following synthetic procedure.

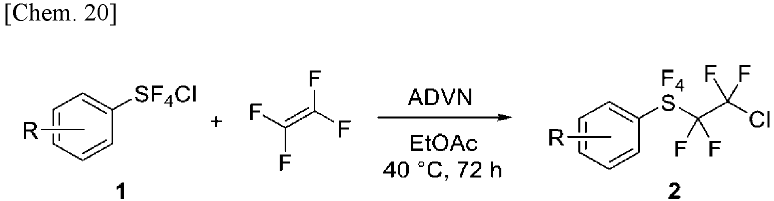

Under a nitrogen atmosphere, a solution obtained by dissolving the compound (1) and ADVN (10% by mole) in EtOAc (0.25 M) was stirred in a microwave vial (25 mL) and then cooled to 0°C. Next, tetrafluoroethylene (TFE) was added to the solution (1.0 atm) by bubbling. After carrying out stirring in an oil bath at 40°C for 72 hours, insoluble solids were removed by filtration, and the filtrate was subjected to evaporation under reduced pressure. The obtained crude product was purified by flash silica gel column chromatography (n-hexane/EtOAc) to obtain the desired compound (2).

### (1) Synthesis of 4-(2-chlorotetrafluoroethyltetrafluoro-λ⁶-sulfanyl)phenyl acetate

According to the procedure described above, 4-(2-chlorotetrafluoroethyltetrafluoro-λ⁶-sulfanyl)phenyl acetate was obtained from 4-(chlorotetrafluoro-λ⁶-sulfanyl)phenyl acetate (0.6 mmol). Following purification carried out by flash silica gel column chromatography (n-hexane/EtOAc = 19/1 (volume ratio)), 4-(2-chlorotetrafluoroethyltetrafluoro-λ⁶-sulfanyl)phenyl acetate (0. 164g, yield: 72%) was obtained as colorless crystals.

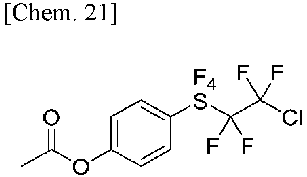

¹H NMR (400 MHz, CDCl₃) δ 7.80 (d, J = 9.2 Hz, 2H), 7.19 (d, J = 8.7 Hz, 2H), 2.32 (s, 3H).

¹⁹F NMR (376 MHz, CDCl₃) δ 49.2 (m, 4F), -67.4 (t, J = 11.6 Hz, 2F), -90.2 (m, 2F).

### (2) Synthesis of 2-(2-chlorotetrafluoroethyltetrafluoro-λ⁶-sulfanyl)pyridine

According to the procedure described above, 2-(2-chlorotetrafluoroethyltetrafluoro-λ⁶-sulfanyl)pyridine was obtained from 2-(chlorotetrafluoro-λ⁶-sulfanyl)pyridine (1.0 mmol). Following purification carried out by flash silica gel column chromatography (n-hexane/EtOAc = 4/1 (volume ratio)), 2-(2-chlorotetrafluoroethyltetrafluoro-λ⁶-sulfanyl)pyridine (0.230 g, yield: 72%) was obtained as a colorless liquid.

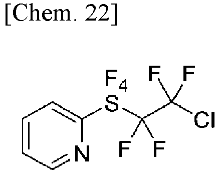

¹H NMR (400 MHz, CDCl₃) δ 8.57 (d, J = 4.1 Hz, 1H), 7.92 (t, J = 7.3 Hz, 1H), 7.77 (d, J = 8.2 Hz, 1H), 7.49 (dd, J = 7.3, 4.6 Hz, 1H).

¹⁹F NMR (376 MHz, CDCl₃) δ 38.0 (m, 4F), -67.5 (m, 2F), -90.7 (m, 2F).

### (3) Synthesis of 2-(2-chlorotetrafluoroethyltetrafluoro-λ⁶-sulfanyl)pyrimidine

According to the procedure described above, 2-(2-chlorotetrafluoroethyltetrafluoro-λ⁶-sulfanyl)pyrimidine was obtained from 2-(chlorotetrafluoro-λ⁶-sulfanyl)pyrimidine (0.5 mmol). Following purification carried out by flash silica gel column chromatography (n-hexane/EtOAc = 1/1 (volume ratio)), 2-(2-chlorotetrafluoroethyltetrafluoro-λ⁶-sulfanyl)pyrimidine (0.129 g, yield: 80%) was obtained as a colorless liquid.

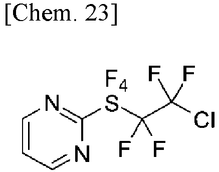

¹H NMR (400 MHz, CDCl₃) δ 8.93 (d, J = 4.6 Hz, 2H), 7.57 (t, J = 4.6 Hz, 1H).

¹⁹F NMR (376 MHz, CDCl₃) δ 33.7 (m, 4F), -67.5 (m, 2F), -91.1 (m, 2F).

### (4) Synthesis of 1-(2-chlorotetrafluoroethyltetrafluoro-λ⁶-sulfanyl)-2,3,4,5,6-pentafluorobenzene

According to the procedure described above, 1-(2-chlorotetrafluoroethyltetrafluoro-λ⁶-sulfanyl)-2,3,4,5,6-pentafluorobenzene was obtained from 1-(chlorotetrafluoro-λ⁶-sulfanyl)-2,3,4,5,6-pentafluorobenzene (0.5 mmol, a mixture of cis form:trans form = 2:3). Following purification carried out by flash silica gel column chromatography (n-hexane/EtOAc = 19/1 (volume ratio)), 1-(2-chlorotetrafluoroethyltetrafluoro-λ⁶-sulfanyl)-2,3,4,5,6-pentafluorobenzene (0.072 g, yield: 58%) was obtained as a colorless liquid.

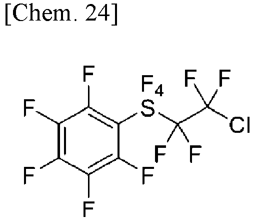

¹⁹F NMR (376 MHz, CDCl₃) δ 59.2 (m, 4F), -67.9 (t, J = 10.8 Hz, 2F), -91.4 (m, 2F), -132.4 (m, 2F), -146.8 (m, 1F), -159.0 (m, 2F).

### [Example 14]

A tetrafluorosulfanyl group-containing aryl compound was synthesized according to the following synthetic procedure.

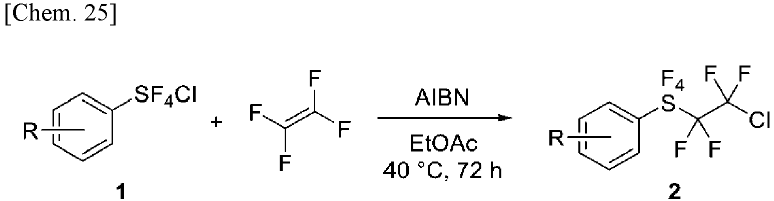

Under a nitrogen atmosphere, a solution obtained by dissolving the compound (1) and AIBN (10% by mole) in EtOAc (0.25 M) was stirred in a microwave vial (25 mL) and then cooled to 0°C. Next, tetrafluoroethylene (TFE) was added to the solution (1.0 atm) by bubbling. After carrying out stirring in an oil bath at 60°C for 48 hours, insoluble solids were removed by filtration, and the filtrate was subjected to evaporation under reduced pressure. The obtained crude product was purified by flash silica gel column chromatography (n-hexane/EtOAc) to obtain the desired compound (2).

### (1) Synthesis of 4-(2-chlorotetrafluoroethyltetrafluoro-λ⁶-sulfanyl)toluene

According to the procedure described above, 4-(2-chlorotetrafluoroethyltetrafluoro-λ⁶-sulfanyl)toluene was obtained from 4-(chlorotetrafluoro-λ⁶-sulfanyl)toluene (1.0 mmol). Following purification carried out by flash silica gel column chromatography (n-hexane/EtOAc = 19/1 (volume ratio)), 4-(2-chlorotetrafluoroethyltetrafluoro-λ⁶-sulfanyl)toluene (0.225 g, yield: 67%) was obtained as colorless crystals.

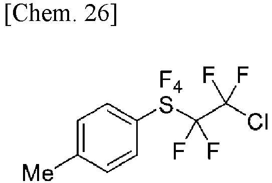

¹H NMR (400 MHz, CDCl₃) δ 7.66 (d, J = 8.7 Hz, 2H), 7.24 (d, J = 8.2 Hz, 2H), 2.40 (s, 3H).

¹⁹F NMR (376 MHz, CDCl₃) δ 48.8 (m, 4F), -67.3 (t, J = 11.6Hz, 2F), -90.2 (m, 2F).

### [Example 15]

A solution obtained by dissolving 2-chlorotetrafluoroethyltetrafluoro-λ⁶-sulfanylbenzene (0.20 mmol) and ADVN (10% by mole) in EtOAc (0.05M) was frozen with liquid nitrogen and degassed under reduced pressure. Next, tetrafluoroethylene (TFE) was added thereto to 1.1 MPa while stirring the solution at 0°C. Stirring was carried out in an oil bath at 40°C for 24 hours to obtain the desired compound (3) in a yield of 42% as determined by NMR. Compound (3) satisfying 1 ≤ n ≤ 7 was detected in the GC/MS analysis.

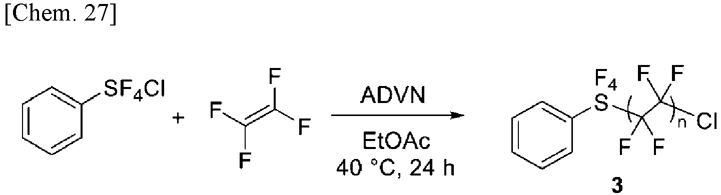

¹⁹F NMR (376 MHz, CDCl₃) δ 48.3-47.3 (m, 4F), -67.9 - -67.3 (m, 2F), -90.5 - - 90.2 (m, 2F), -121.5 - -118.4 (m, 4(n-1)F).

### [Example 16]

To a solution obtained by dissolving 3-(2-chlorotetrafluoroethyltetrafluoro-λ⁶-sulfanyl)nitrobenzene (0.75 mmol) in ethanol (15 mL), iron (10 equivalents) and a saturated aqueous ammonium chloride solution (3.7 mL) were added, and stirring was carried out in an oil bath at 80°C for 2 hours. Thereafter, insoluble solids were removed by filtration through celite, and the filtrate was subjected to evaporation under reduced pressure. The obtained crude product was dissolved in ethyl acetate and washed with water, and the aqueous layer was extracted twice with ethyl acetate. Then, the combined organic extracts were dried with sodium sulfate, and the solvent was evaporated under reduced pressure. The obtained crude product was purified by flash silica gel column chromatography (n-hexane/EtOAc = 4/1 (volume ratio)), whereby the desired 3-(2-chlorotetrafluoroethyltetrafluoro-λ⁶-sulfanyl)aniline (0.209g, yield: 83%) was obtained as a pale yellow liquid.

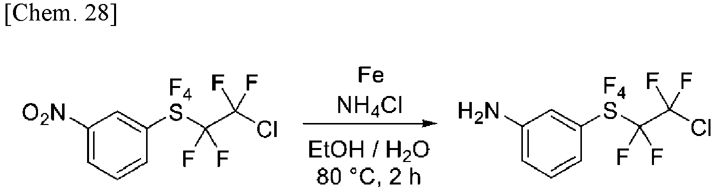

¹H NMR (400 MHz, CDCl₃) δ 7.20 (t, J = 7.8 Hz, 1H), 7.14 (d, J = 8.2 Hz, 1H), 7.07 (t, J = 2.0 Hz, 1H), 6.76 (d, J = 7.8 Hz, 1H), 3.84 (br, 2H).

¹⁹F NMR (376 MHz, CDCl₃) δ 48.0 (m, 4F), -67.3 (t, J = 10.0 Hz, 2F), -90.3 (m, 2F).

### [Example 17]

Under a nitrogen atmosphere, 3-(2-chlorotetrafluoroethyltetrafluoro-λ⁶-sulfanyl)aniline (0.40 mmol), 1,1'-azobis(cyclohexane-1-carbonitrile) (V-40) (15% by mole), and tris(trimethylsilyl)silane (5.0 equivalents) were dissolved in toluene (2.0mL) in a microwave vial (10 mL), and stirring was carried out in an oil bath at 100°C for 2 hours. Thereafter, the solvent was evaporated under reduced pressure, the obtained crude product was dissolved together with dimethylcarbamoyl chloride (6.0 equivalents) and triethylamine (2.0 equivalents) in tetrahydrofuran (2.0 equivalents), and then stirring was carried out at room temperature for 20 hours. The obtained crude product was extracted three times with a mixed solvent of ethyl acetate/hexane and then washed three times with each of a saturated aqueous citric acid solution and a saturated aqueous sodium chloride solution. The obtained organic extract was dried with sodium sulfate, the solvent was evaporated under reduced pressure, and then purification was carried out by flash silica gel column chromatography (n-hexane/EtOAc = 2/1 (volume ratio)) to obtain the desired N,N-dimethyl-N'-[3-(1,1,2,2-tetrafluoroethyltetrafluoro-λ⁶-sulfanyl)phenyl]urea (0.045 g, yield: 30%) as a pale yellow liquid.

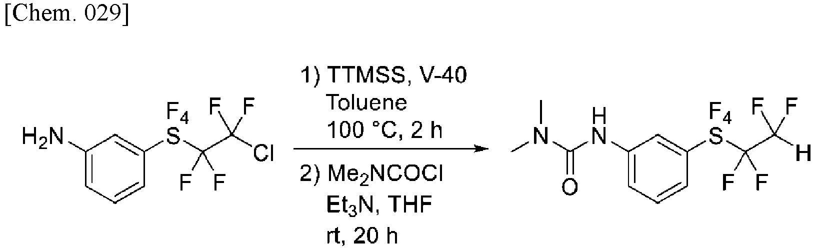

¹H NMR (400 MHz, CDCl₃) δ 7.83 (m, 1H), 7.52 (m, 1H), 7.38 (m, 1H), 7.30 (m, 1H), 6.70 (br, 1H), 6.14 (m, 1H), 3.00 (s, 6H).

¹⁹F NMR (376 MHz, CDCl₃) δ 43.4 (m, 4F), -97.5 (t, J = 10.0 Hz, 2F), -133.6 (d, J = 55.2 Hz, 2F).

### [Example 18]

4-(2-chlorotetrafluoroethyltetrafluoro-λ⁶-sulfanyl)bromobenzene (0.50 mmol) was dissolved together with tetrakis(triphenylphosphine) palladium(0) (3% by mole) in dimethoxyethane (1.0 mL), and stirring was carried out for 10 minutes. Thereafter, a saturated ethanol solution of phenylboronic acid (1.1 equivalents) and a saturated aqueous solution of sodium carbonate (2.0 equivalents) was added thereto, and stirring was carried out in an oil bath at 80°C for 18 hours. Thereafter, insoluble solids were removed by filtration, and the filtrate was dissolved in ethyl acetate and then washed three times with a saturated aqueous sodium chloride solution. The obtained organic extract was dried with sodium sulfate, and the solvent was evaporated under reduced pressure. Then, purification was carried out by flash silica gel column chromatography (n-hexane/EtOAc = 19/1 (volume ratio)) to obtain the desired 4-(2-chlorotetrafluoroethyltetrafluoro-λ⁶-sulfanyl)biphenyl (0.182g, yield: 92%) was obtained as a colorless solid.

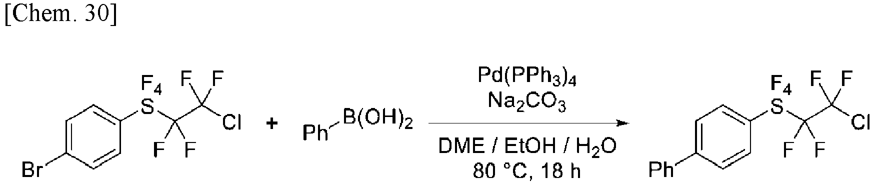

¹H NMR (400 MHz, CDCl₃) δ 7.87 (d, J = 8.2 Hz, 2H), 7.66 (d, J = 8.2 Hz, 2H), 7.60 (d, J = 8.2 Hz, 2H), 7.50 (t, J = 7.8 Hz, 2H), 7.44 (t, J = 7.8 Hz, 1H).

¹⁹F NMR (376 MHz, CDCl₃) δ 48.9 (m, 4F), -67.3 (t, J = 11.6 Hz, 2F), -90.1 (m, 2F).

### [Industrial Applicability]

The present invention provides a manufacturing method that makes it possible to synthesize an SF₄-containing aryl compound in a single step under relatively mild conditions. The present invention is useful for introducing an SF₄ group into active ingredients of pharmaceutical drugs or agricultural chemicals or in organic materials.

## Claims

1. A method for producing a tetrafluorosulfanyl group-containing aryl compound, comprising:
subjecting a thioaryl compound represented by General Formula (2) to a reaction by adding to an olefin compound represented by General Formula (3) in the presence of a radical initiator to synthesize a tetrafluorosulfanyl group-containing aryl compound represented by General Formula (1),
[Chem. 1]
A¹-SF₄Cl (2)
[in the formula, A¹ is an aryl group which may have a substituent, or a heteroaryl group which may have a substituent] 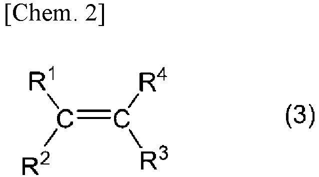
[in the formula, R¹, R², R³, and R⁴ are each independently a hydrogen atom, a fluorine atom, or a chlorine atom, provided that two or more of R¹, R², R³, and R⁴ are a fluorine atom] 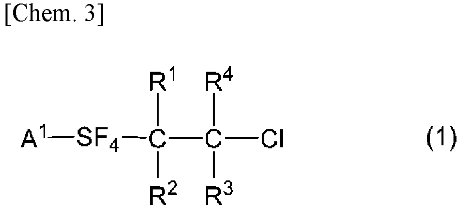
[in the formula, A¹, R¹, R², R³, and R⁴ are the same as above].

2. The method for producing a tetrafluorosulfanyl group-containing aryl compound according to Claim 1,
wherein A¹ is an aryl which may have one or more kinds of substituents selected from the group consisting of a halogen atom, an alkyl group, an alkenyl group, an aryl group, an alkoxy group, a hydroxy group, a carboxy group, an acyl group, a cyano group, an amino group, and a nitro group.

3. The method for producing a tetrafluorosulfanyl group-containing aryl compound according to Claim 1 or 2,
wherein the reaction is carried out at -40°C to 130°C.

4. A tetrafluorosulfanyl group-containing aryl compound represented by General Formula (1), 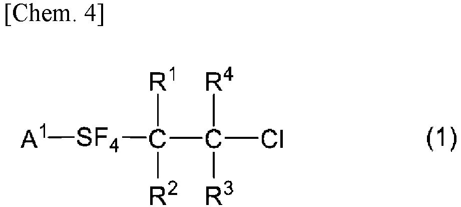 [in the formula, A¹ is an aryl group which may have a substituent, or a heteroaryl group which may have a substituent; and R¹, R², R³, and R⁴ are each independently a hydrogen atom, a fluorine atom, or a chlorine atom, and two or more of R¹, R², R³, and R⁴ are a fluorine atom].
